Europäisches Patentamt

European Patent Office

Office européen des brevets

(19)

(11) **EP 1 150 693 B1**

(12) **FASCICULE DE BREVET EUROPEEN**

(45) Date de publication et mention
de la délivrance du brevet:
**24.03.2004 Bulletin 2004/13**

(51) Int Cl.⁷: **A61K 35/14**, A61K 9/127,
A61K 48/00, A61K 39/42,
A61P 31/18, A61P 37/00
// (C07K14/16, A61K39:21,
C12N5/10)

(21) Numéro de dépôt: **00905141.8**

(22) Date de dépôt: **15.02.2000**

(86) Numéro de dépôt international:
**PCT/FR2000/000375**

(87) Numéro de publication internationale:
**WO 2000/047216 (17.08.2000 Gazette 2000/33)**

(54) **COMPOSITIONS IMMUNOGENES UTILISABLES COMME VACCINS**

IMMUNOGENE ZUSAMMENSETZUNGEN VERWENDBAR ALS IMPFSTOFFE

IMMUNOGENIC COMPOSITIONS FOR USE AS VACCINES

(84) Etats contractants désignés:
**AT BE CH CY DE DK ES FI FR GB GR IE IT LI LU
MC NL PT SE**

(30) Priorité: **15.02.1999 FR 9901794**

(43) Date de publication de la demande:
**07.11.2001 Bulletin 2001/45**

(73) Titulaire: **Institut de Recherche pour le
Développement ( IRD)
75480 Paris Cedex 10 (FR)**

(72) Inventeur: **VEAS, Francisco
F-34130 Mauguio (FR)**

(74) Mandataire: **Peaucelle, Chantal et al
Cabinet Armengaud Aine
3, avenue Bugeaud
75116 Paris (FR)**

(56) Documents cités:
**WO-A-97/26009**

- **R. LACASSE ET AL.: "Fusion-competent
vaccines: Broad neutralization of primary
isolates of HIV." SCIENCE, vol. 283, no. 5400, 15
janvier 1999 (1999-01-15), pages 357-362,
XP002120812 Washington, DC, tats-Unis cité
dans la demande**

- **J. ROSSIO ET AL.: "Inactivation of human
immunodeficiency virus type 1 infectivity with
preservation of conformational and functional
integrity of virion surface proteins." JOURNAL
OF VIROLOGY, vol. 72, no. 10, octobre 1998
(1998-10), pages 7992-8001, XP002120813
Baltimore, MD, tats-Unis**

- **D. MONTEFIORI ET AL.: "Magic of the occult?"
SCIENCE, vol. 283, no. 5400, 15 janvier 1999
(1999-01-15), pages 336-337, XP002120814
Washington, DC, tats-Unis**

- **D. CHAN ET AL.: "HIV entry and its inhibition."
CELL, vol. 93, no. 5, 29 mai 1998 (1998-05-29),
pages 681-684, XP002120815 Cambridge, MA,
tats-Unis**

- **K. SCHÖNNING ET AL.: "Frosne
fusionskomplekser - en genvej til en
HIV-vaccine? (Frozen fusion complexes - a
shortcut to a HIV-vaccine?)" UGESKRIFT FOR
LAEGER, vol. 161, no. 31, 2 août 1999
(1999-08-02), pages 4415-4416, XP002120816
Copenhague, Danemark**

- **Q. SATTENTAU ET AL.: "Antibody neutralization
of HIV-1 and the potential for vaccine design."
IMMUNOLOGY LETTERS, vol. 66, no. 1-3, mars
1999 (1999-03), pages 143-149, XP002120817
Amsterdam, Pays-Bas**

## Description

L'invention a pour objet des compositions immunogènes utilisables, notamment, comme vaccins, chez les mammifères, contre des pathologies infectieuses.

Les pathologies visées conformément à l'invention sont du type cellule-dépendant c'est-à-dire que le processus infectieux se développe après la liaison, suivie ou non de la fusion, de l'agent pathogène à une cellule cible de mammifère.

On connaît le rôle majeur joué par certaines régions de ces agents pathogènes dans les infections qu'ils provoquent.

Ainsi, en ce qui concerne par exemple VIH, divers travaux ont montré que les régions impliquées dans les interactions avec les récepteurs des cellules cibles (récepteur CD4 et récepteurs de chimiokines, comme CCR5 et CXCR4) sont des régions conservées de l'enveloppe du virus.

La formation d'anticorps contre ces régions se heurte jusqu'à présent au problème de l'accès aux épitopes d'intérêt. Des interactions et changements structurels complexes interviennent durant la liaison du virus à la cellule cible, puis la fusion, qui empêche en effet l'accès aux épitopes des régions fusionnées et rend difficilement accessibles ceux des régions voisines de celles directement impliquées dans l'interaction avec les cellules cibles, dont l'exposition de surcroît peut être de courte durée.

Avec un système expérimental décrit dans Science, vol. 283, 15 janvier 1999, LaCasse et al ont probablement obtenu chez la souris, la formation d'anticorps neutralisants vis-à-vis d'isolats infectieux de VIH, correspondant donc à un certain degré d'accessibilité desdits épitopes.

Le système proposé est obtenu par fusion de fibroblastes simiens, modifiés pour exprimer l'enveloppe fonctionnelle d'un isolat primaire de VIH-1 (P168), avec des neuroblastomes humains exprimant le récepteur CD4 et le co-récepteur CCR5, puis fixation du complexe formé, 4 à 5 h après le début de la fusion, avec du formaldéhyde. Dans des essais sur la souris, un tel complexe s'est montré capable de former des anticorps neutralisants vis-à-vis d'isolats infectieux de VIH provenant d'un très grand éventail de sous-types.

Toutefois, un tel système n'est pas utilisable chez l'homme compte tenu du danger potentiel des populations cellulaires formant le complexe immunogène.

De plus, les conditions mises en oeuvre pour élaborer le système ne garantissent pas la préservation de la conformation des épitopes et l'accès à une large région d'intérêt.

En contrôlant la progression de la fusion et les conditions de réalisation de la fixation, les inventeurs ont obtenu des complexes à un stade où les épitopes d'intérêt, y inclus des épitopes au voisinage de ceux mis en jeu dans la fusion, sont exposés de manière particulièrement satisfaisante et doivent être préservés dans leur conformation naturelle. Les propriétés immunogènes mises en évidence chez de tels complexes en font alors des candidats de choix pour l'élaboration de vaccins, présentant l'avantage considérable d'être utilisables chez l'homme lorsqu'on met en oeuvre, pour la préparation des complexes, des produits appropriés.

De manière avantageuse, de tels systèmes s'avèrent d'application générale pour tout type d'agent pathogène avec un processus infectieux impliquant une étape de liaison, suivie ou non de fusion avec les cellules cibles.

L'invention a donc pour but de fournir des compositions immunogènes et des compositions vaccinales, utilisables chez les mammifères, contre les pathologies infectieuses, dépendantes d'une infection cellulaire.

Elle vise également les complexes immunogènes mis en oeuvre dans ces compositions, en tant que nouveaux produits, ainsi que les anticorps formés contre les épitopes des régions de l'agent pathogène dans le voisinage et même à proximité immédiate de celles impliquées dans la fusion avec la cellule cible.

Les compositions immunogènes selon l'invention sont caractérisées en ce qu'elles sont élaborées à partir de préparations obtenues par

**.** incubation de premiers moyens, exprimant le et/ou les récepteurs cibles d'un agent pathogène infectieux tel que défini ci-dessus, avec des seconds moyens exprimant au moins les régions de l'agent pathogène reconnaissant lesdites cibles, dans des conditions permettant l'interaction des premiers et seconds moyens de manière à former un complexe, cette étape d'incubation étant réalisée selon des durées différentes, afin de conduire à des complexes correspondant à différents stades de fusion et présentant donc des expositions et conformations différentes d'épitopes nouvellement démasqués, et

**.** mise en contact des complexes formés avec un agent fixateur, pendant des durées différentes, de manière à fixer des complexes avec différentes expositions et conformations des épitopes contre lesquels on souhaite former des anticorps,

lesdits premiers et seconds moyens étant choisis parmi des produits tolérés par les mammifères.

Selon un mode de réalisation de l'invention, lesdits premiers moyens sont des cellules autologues de mammifères. Il s'agit de cellules saines provenant du mammifère à vacciner, en particulier de l'homme par exemple des PBMC totaux, des lymphocytes ou des macrophages isolés.

Ces cellules sont stimulées si nécessaire de manière à exprimer, en quantité suffisante, le ou les récepteurs nécessaires pour l'interaction recherchée.

Dans un autre mode de réalisation de l'invention, lesdits premiers moyens sont des vecteurs exprimant le ou les récepteurs cibles à leur surface. De tels vecteurs comprennent par exemple des vecteurs viraux comme le *baculovirus*, le virus de la forêt de Semliki (SFV), ou encore des levures comme *Saccharomyces cerevisae*.

Selon encore un autre mode de réalisation de l'invention, lesdits premiers moyens sont des liposomes portant à leur surface le ou les récepteurs cibles correctement présentés, mimant ainsi les cellules cibles.

Les récepteurs transmembranaires (à un ou plusieurs passages) cibles seront inclus dans les liposomes à partir de récepteurs exprimés en grandes quantités, grâce à des vecteurs d'expression, à la surface de cellules ($10^6$ à $10^7$/cellule) soit d'insecte, de levure ou de mammifère. Le passage de récepteurs de cellules aux liposomes se fait après isolement de membranes cellulaires, leur traitement avec un détergent approprié selon des protocoles définis par J.L. Rigaud (voir référence ci-après) et finalement leur inclusion dans les membranes appropriées.

Conformément à l'invention, les seconds moyens mis en oeuvre sont ceux exprimant au moins les régions de l'agent pathogène infectieux capables de se lier aux cellules cibles et de fusionner avec ces cellules.

Des seconds moyens mis en oeuvre selon l'invention sont ainsi constitués par des cellules préalablement transformées avec un vecteur portant au moins une région de liaison à au moins un récepteur cible. Il s'agit avantageusement de vecteurs viraux tels qu'envisagés ci-dessus, à savoir le *baculovirus*, SFV, ou encore des levures comme *Saccharomyces cerevisae*.

En variante, les seconds moyens sont constitués par les vecteurs viraux eux-mêmes.

Dans une autre variante, les seconds moyens sont des cellules infectées produisant les agents pathogènes ou sont constitués par les agents pathogènes infectieux eux-mêmes.

Les agents pathogènes évoqués ci-dessus peuvent être des virus, et notamment des rétrovirus, des bactéries, des mycobactéries, ou des parasites comme *Plasmodium sp, Leishmania sp, Trypanosoma cruzi* et *Trypanosoma brucei.*

L'invention présente un intérêt tout particulièrement dans le cas de VIH, ce terme étant utilisé dans la description et les revendications pour désigner aussi bien les isolats des différentes souches humaines ou animales, ainsi que le virus avec une enveloppe naturelle ou recombinante, le cas échéant mutée.

L'invention vise ainsi en particulier les compositions immunogènes dans lesquelles lesdites préparations sont obtenues par incubation de premiers moyens exprimant le récepteur CD4 et/ou des co-récepteurs du VIH, avec des seconds moyens exprimant au moins les régions conservées des protéines d'enveloppe gp120 ou gp160.

L'expression "protéine d'enveloppe" telle qu'utilisée dans la description et les revendications englobe aussi bien la protéine naturelle que les protéines recombinantes telles que connues de l'homme du métier, ou des protéines mutées. Ces dernières présentent l'avantage de permettre l'identification des sites de l'enveloppe impliqués dans la reconnaissance par les anticorps, tels qu'ils sont produits après leur fusion avec les récepteurs cibles.

Dans de telles compositions, les premiers moyens mis en oeuvre sont constitués par des cellules autologues de mammifères comme indiqué plus haut. Ces cellules sont stimulées de manière à exprimer, en quantité suffisante pour l'interaction recherchée, le récepteur CD4 et/ou des co-récepteurs de CD4, comme CCR5, CXCR4, la protéine bande 3 ou d'autres protéines transmembranaires. La stimulation est réalisée par exemple avec PHA et/ou IL2.

En variante, lesdits premiers moyens sont des vecteurs viraux exprimant à leur surface CD4 et/ou des co-récepteurs de VIH. Comme indiqué ci-dessus, de tels vecteurs comprennent le *baculovirus*, SFV, ou encore des levures comme *Saccharomyces cerevisae*.

Dans encore une autre variante, lesdits premiers moyens sont des liposomes exprimant à leur surface le récepteur CD4 et/ou des co-récepteurs comme indiqué plus haut. Ces liposomes peuvent ainsi comporter le récepteur CD4 et/ou des co-récepteurs du VIH.

Dans de telles compositions comprenant avantageusement l'un des premiers moyens considérés ci-dessus, les seconds moyens mis en oeuvre expriment au moins les régions conservées des protéines d'enveloppe gp120 ou gp160.

Ces seconds moyens sont constitués par des cellules préalablement transformées avec un vecteur d'enveloppe virale de VIH, ou tout au moins des régions conservées des protéines gp120 ou gp160. Il s'agit avantageusement de vecteurs viraux tels qu'envisagés ci-dessus.

En variante, les seconds moyens sont constitués par les vecteurs viraux eux-mêmes.

Dans une autre variante, les seconds moyens sont des cellules infectées produisant VIH ou sont constitués par le virus VIH lui-même. On met avantageusement en oeuvre un virus fusogène, provenant d'isolats primaires.

On rappelle que, conformément à l'invention, les protéines gp120 ou gp160, ou les protéines comportant au moins les régions conservées de protéines gp120 ou gp160 sont sous forme naturelle, ou sous forme recombinantes, ou sous forme mutée.

De manière avantageuse, lesdits seconds moyens sont constitués par de telles protéines et comprennent donc une gp120 soluble monomère, le cas échéant sous forme recombinante ou un oligomère de gp120 ou gp160, également le cas échéant sous forme recombinante. Il peut s'agir également des parties de ces protéines comportant au moins les régions conservées.

Dans un mode avantageux de réalisation de l'invention, les seconds moyens comprennent un anticorps monoclonal anti-co-récepteur. Il s'agit par exemple de l'anticorps monoclonal 17b, 48d ou CG10. Cette disposition permet d'accéder à des épitopes d'intérêt au voisinage du site de liaison au co-récepteur sur la gp120.

Des compositions préférées de ce type se présentent sous forme moléculaire et comprennent le CD4 soluble comme premier moyen, la gp120 soluble monomère comme second moyen, et un anticorps monoclonal comme défini ci-dessus, ou un fragment Fab d'un tel anticorps.

L'incubation des premiers et seconds moyens est réalisée de manière à former un complexe dans lequel les premiers moyens sont engagés dans un processus de fusion avec les seconds moyens.

Il est avantageux de réaliser cette étape selon des durées différentes, ce qui permet de disposer de différents stades de fusion et de choisir, par test sur l'animal, le stade qui donnera les meilleurs résultats par rapport aux propriétés immunogènes recherchées.

On opère le plus généralement sur des durées variant de 15 min à 5 h.

Les conformations de ces différents stades sont fixées par addition d'un agent capable d'arrêter la fusion sans dénaturer significativement les épitopes d'intérêt.

Un agent fixateur tout particulièrement préféré à cet égard est constitué par la 2,2'-dithiopyridine (aldidrithiol-2 ou, en abrégé, AT-2)

D'autres agents peuvent être utilisés notamment lorsqu'on vise des applications en recherche, comme par exemple le formol.

La fixation est réalisée sur des durées différentes ce qui permet d'étudier les effets de la cinétique de fixation sur l'immobilisation des épitopes présentés.

Les préparations obtenues sont récupérées, lavées et mise en suspension dans un tampon approprié.

On évalue ensuite sur l'animal les meilleurs temps de fusion et de fixation pour induire la meilleure réponse immune et générer les anticorps qui empêchent l'infection.

L'invention vise en tant que nouveaux produits les complexes antigéniques résultant des étapes de fusion et fixation.

Ces complexes, plus spécialement sous forme cristallisée, permettent de manière avantageuse d'étudier et de mettre en évidence les sites d'interaction de la gp120 et/ou de la gp41 de l'enveloppe, mais aussi les régions à proximité immédiate de ces sites.

Le procédé de préparation des compositions immunogènes définies ci-dessus entre également dans le champ de l'invention. Ce procédé comprend la mise en oeuvre desdits premiers et seconds moyens, leur fusion et leur fixation, comme défini ci-dessus.

L'étude des propriétés immunologiques des compositions de l'invention a permis de mettre en évidence leur fort pouvoir immunogène.

Ainsi, les sérums recueillis après administration de ces compositions à des souris transgéniques CD4+, CXCR5+, ou à des lapins a montré des taux élevés en anticorps.

Ces sérums, ainsi que les anticorps obtenus à partir de ces sérums, puis purifiés selon les techniques classiques, font partie de l'invention.

Ces anticorps sont ainsi caractérisés en ce qu'ils sont capables de reconnaître, selon une réaction de type antigène-anticorps, un agent pathogène infectieux et d'inhiber ainsi son pouvoir infectieux.

Comme le montrent les expériences réalisées *in vivo* sur les mammifères, les sérums et anticorps purifiés sont capables d'inhiber l'infectivité d'un large spectre d'isolats primaires de VIH.

L'invention vise donc des compositions vaccinales caractérisées en ce qu'elles renferment une quantité efficace de compositions immunogènes telles que définies ci-dessus avec un véhicule, inerte, acceptable pour l'administration à un mammifère, en association le cas échéant avec un adjuvant.

Comme adjuvant capable d'augmenter les réactions immunitaires de l'organisme du mammifère à vacciner, on citera les adjuvants minéraux comme le phosphate d'aluminium, les adjuvants huileux comme l'adjuvant incomplet de Freund, les adjuvants d'origine bactérienne comme l'adjuvant complet de Freund. Un adjuvant particulièrement préféré est constitué par l'adjuvant Ribi.

La composition vaccinale est administrée par voie injectable, ou encore par voie orale, avec un rappel 3 mois après l'injection.

Les compositions vaccinales sont administrées en une quantité et selon un protocole permettant de conférer à l'hôte une immunité à l'égard des antigènes de l'agent pathogène infectieux.

D'autres caractéristiques et avantages de l'invention sont donnés dans les exemples qui suivent :

Préparation d'une composition immunogène cellulaire.

On utilise d'une part des cellules humaines autologues prélevées sur le patient à vacciner et exprimant CD4/CCRS et CXCR4 après stimulation pendant 3 à 6 jours avec PHA/IL2 en opérant selon la méthode de Riley et al, JVI 1998,71 : 8273-8280, d'autre part un isolat primaire de VIH-1 ou des cellules infectées par un tel isolat.
On répartit ces 2 populations dans différentes boîtes de pétri pour une incubation à 37° C pendant des durées différentes respectivement de 15, 30, 45, 60, 120, 180, 240 et 300 min. Pour chaque expérience, on utilise 3 x $10^6$ à $10^8$ cellules autologues et par exemple l'isolat primaire 92 HT 593 ou ACH168.10 provenant de Aids Research and Reference Reagent Programm, NIH (EUA), lesquels utilisent les deux co-récepteurs CXCR4 et CCR5 de VIH.
Au terme de la durée prévue, on ajoute l'AT-2 à raison de 600 à 1000 µm, en solution dans PBS. La fixation est réalisée à 4°C pendant un temps comme recommandé par Rossio et al, JVI, 1998, <u>72</u>, 7992, ou 1 à 12 h. Les complexes cellulaires formés sont lavés avec PBS, récupéré et remis en suspension à raison de $10^6$ à $10^8$ complexe cellulaire/0,1 ml dans PBS/(DM50 10%) afin de les conserver à -80°C.
L'immunogène congelé est décongelé et lavé plusieurs fois, puis mis en présence de la même quantité d'un adjuvant, par exemple Ribi (R-700 ou R-730).

Préparation d'une composition immunogène avec des vecteurs viraux.

On opère comme indiqué dans l'exemple 1, mais on utilise d'une part un système *baculovirus* portant les récepteurs CD4 et CCRS et/ou CXCR4 à sa surface, d'autre part le virus Vaccinia ou le virus de la forêt de Semliki portant la gp120 monomère ou l'oligomère de gp120 et gp41. Dans d'autres expériences, on utilise un système baculovirus permettant l'expression de protéines dans sa propre membrane (Boublick et al, Biotechnology, 1995, 13: 1079-84).

Préparation d'une composition immunogène à base de lisposomes.

On opère comme indiqué dans l'exemple 1, mais on utilise des liposomes portant à leur surface CD4 et CCRS et/ou CXCR4. Pour préparer ces liposomes, on a recours aux enseignements de Rigaud et al dans Biochim. Acta Phys. 1995, 1231:223-246 ou de Pitard et al dans Eur. J. Biochem. 1996, 235, 3769-3778, qui décrivent des liposomes portant des protéines membranaires ou transmembranaires fonctionnelles. On les met à incuber avec les enveloppes de VIH-1 ou de VIH-2 aux fins de fusion. Il s'agit soit de virus VIH avec différentes enveloppes recombinantes (utilisation de virus complémentés pseudotypés), soit de vecteurs viraux portant les enveloppes recombinantes de VIH. Pour le passage de récepteurs transmembranaires de membranes cellulaires aux liposomes, on a recours à des vecteurs à forte expression comme le virus *Vaccinia*, le *baculovirus*, ou *Saccharomyces cerevisae* permettant l'obtention de 10° à $10^7$ copies de récepteur par cellule.

Préparation vaccinale administrée à un mammifère, contre une infection par VIH-1

On utilise une composition immunogène selon l'exemple 3, préalablement testée sur l'animal pour évaluer sa capacité à produire une réponse d'anticorps. Cette composition est lavée à plusieurs reprises dans PBS, mise en suspension dans du sérum physiologique, puis additionnée à cette composition, à titre d'adjuvant, de Ribi.
La préparation est injectée à raison de 0,05 ml à 1 ml à des animaux expérimentaux (souris, singe, homme, selon le cas). On procède à un rappel 4 à 6 mois plus tard.

**Clonage et expresion du co-récepteur CCR5 et/ou du récepteur CD4 à la surface de cellules d'insectes.**

**Clonage du CCR5/6Histidine et expression de baculovirus**

1. Le pcDNA3 CCR5 contient la séquence complète du CCR5 humain (n° d'accession à Genbank : NM 000579). On amplifie par PCR la région C-terminale entre le site Eco-R1 et TGA. A partir de l'ATG (+10) le site EcoRI est en position 793 et le codon Stop est en position 1066.
2. Ce fragment est cloné dans un plasmide pUC18, pour séquençage, puis le fragment est reinséré dans pcDNA3 CCR5 en EcoRI-XbaI.
3. Ainsi le plasmide pcDNA3 CCR5 est modifié et devient pcDNA3 CCR5-6HIS.
4. Le gène CCR5 plus son tag 6HIS en C-terminal est extrait du pcDNA3 par coupure BamHI-BamHI.
5. Le gène CCR5-6HIS est alors introduit par BgI II-bg1 I en aval du promoteur p10 du vecteur de transfert (baculo) p 119.

6. Une fois que le vecteur de transfert p119 a té additionné du gène CCR5-6HIS, il est co-transfecté avec de l'ADN du virus AcSLP10 dans les cellules Sf9 d'insecte *(Autographa californica)*.

7. L'expression de CCR5-6HIS est obtenue à la surface de ces cellules. La densité de ces CCR5-6HIS est évaluée par scatchard (REF).

8. La caractérisation fonctionnelle de ce récepteur est évaluée par la capacité de virus VIH ou des gp120 de VIH à se fixer à ces cellules.

**Clonage du CD4. On procéde selon un protocole de même type :**

1. Le plasmide pGEM-T contenant le gène CD4 est additionné du tag 6 Histidine. A partir de l'ATG (+1) le site Bsu361 est en position 1087 et le codon Stop en position 1375.

2. Le gène CD4 additionné de 6 histidines en C-terminal est introduit an aval du promoteur de la polyédrine dans le vecteur de transfert pGEMAc116T par BgI II-bgI I.

3. Le vecteur de transfert pGEMAc116T CD4-6 HIS sera co-transfecté avec l'ADN du virus AcSLP10 dans les cellules Sf9 d'insecte *(Autographa californica)*

4. L'expression du CD4-6HIS est obtenue à la surface de ces cellules. La densité de ces CCR5-6HIS est évaluée par scatchard (Cahoreau et al, 1992, Biochimie, 74, 1053-1065).

5. La caractérisation fonctionnelle de ce récepteur est évaluée par la capacité de virus ou des gp120 de VIH à se fixer à ces cellules.

**Expression du double recombinant CD4 et du CCR5 :**

1. Les gènes CCR5-6 HIS et CD4-6 HIS sont respectivement clonés dans les vecteurs p119 et pGEMAc116T.

2. L'expression du CCR5-6HIS sous contrôle du promoteur p10 et du CD4-6 HIS est effectuée sous contrôle du promoteur de la polyédrine

3. Les 2 vecteurs de transfert seront co-transfectés avec l'ADN du virus AcSLP10 dans les cellules Sf9d'insecte *(Autographa californica)*.

4. On obtient ainsi des cellules Sf9 exprimant à leur surface un double recombinant CCR5-6HIS et CD4-6HIS dans des proportions égales.

Purification des membranes cellulaires enrichies en CCR5 ou CD4 et préparation des protéoliposomes correspondants.

A partir de membranes cellulaires Sf9, il est ainsi possible de reconstituer les récepteurs dans des liposomes contenant :

1. seulement du CCR5,
2. seulement du CD4
3. du CCR5 et du CD4 dans les proportions choisies à partir des cellules exprimant séparément CCR5 et CD4.
4. du CCR5 et CD4 dans de proportions choisies à partir des cellules exprimant en même temps et dans des quantités identiques CCR5 et CD4.

Il s'agit d'obtenir des enveloppes de VIH fusionnant avec le co-récepteur de VIH, puis d'arrêter ces fusions au moyen d'un fixateur comme le paraformaldéhyde ou le glutaraldéhyde et d'injecter ce couple immunisant aux souris transgéniques huCD4/huCCR5 ou à des modèles macaques ou d'autres simiens. En fonction des résultats, les préparations peuvent alors être injectées chez l'homme.

Le même système est mis en place pour CXCR4.

**Stratégies pour la reconstitution de protéines transmembranaires dans des protéoliposomes**

Les cellules SF9 *d'Autographa californica*, qui surrexpriment les récepteurs CCR5 (ou CXCR4) et/ou CD4 seront digérées par détergents appropriés dans le but d'obtenir des protéoliposomes selon une méthode dérivée de Rigaud et al, 1988, Biochemistry, 27, 2677-2688 ; Paternostre et al, Biochemistry 1988, 27, 2668-2677 ; Gaymard et al, J. Biol. Chem. 1996, 271, 22863-22870.

*Evaluation des capacités fonctionnelles du CCRS ou/et du CD4*

1. exprimés à la surface de cellules Sf9

a) Présence de récepteurs à la surface cellulaires analysé par FACS et microscopie confocale :

I. Avec des anticorps spécifiques anti-CD4 ou anti-CCR5
II. Avec des gp 120 marqués avec des anticorps spécifiques
III.Avec du VIH-1 portant les enveloppes mutées ou non
IV.Dans un premier temps on caractérise la fonctionnalité des récepteurs à la surface de cellules Sf9 et on quantifie par scatchard (Cahoreau et al ci-dessus) le nombre de molécules par cellule dont nous connaissons l'environnement lipidique des membranes cellulaires.

b) Analyse confocale de fluorescence spécifique de la fusion par des méthodes dérivées de Robert Blumenthal (NIH, communication personnelle, 2000) et par celle de Vidal et al, 1996, J.Biol. Chem. 270, 17823-17829.

I. Après contact avec des cellules exprimant l'enveloppe de VIH-1 (mutée ou non)
II. Après contact avec des VIH-1 (ou des pseudotypes viraux portant les enveloppes mutées ou non)
III.Avec des pseudo-particules virales.

2 Dans les protéoliposomes correspondants

a. Analyse confocale de fluorescence spécifique par les méthodes ci-dessus
b. D'autres méthodes de transfert d'énergie (FRET : fluorescence résonance energy transfer, (Mattjus et al, 1999, Anal. Biochem. 268,297-304).

**CCR-5, Introduction de 6 résidus Histidine en C-terminal**

**1- Amplification par PCR de la région C-terminale entre le site EcoRI et le TGA pour CCR5 ;**

5'                                                    3'
CCT TCC AGG AAT TCT TTG GCC

**Bac-CCR5** : introduction dans cet oligonucléotide d'un site StuI (créé grâce à la dégénérescence du code génétique) et d'un site XbaI pour le réintégration du fragment muté dans le plasmide d'origine.

```
        val     gly      leu   opa

        GTG    GGC      TTG  TGA~
        GTC    GGA      TTA
        GTA    GGT      CTA
        GTT    GGG      CTG
                         CTC
                         CTT
                 StuI                        XbaI
     5'                                              3'
       G GAA ATA TCT GTA GGC CTG TGA CAT CTA GAG GTG
       C CTT TAT AGA C AT CCG GAC ACT GTA GAT CTC CAC
     3'                                              5'
       ------------------------------------------+++++++++++++
            appariées                    non appariées
```

Le fragment EcoRI-XbaI amplifié est cloné dans un vecteur pUC en EcoRI-XbaI, puis séquencé. Le fragment muté est ensuite réinséré dans le plasmide d'origine en EcoRI-XbaI.

## 2 - Introduction des 6 codons histidine en aval du C-terminal du CCR5.

Le plasmide ainsi modifié est coupé par StuI et Xba puis ligué avec le fragment d'ADN StuI-XbaI décrit ci-dessous. Ce fragment apporte 6 codons Histidine et un codon Stop TAA.

```
  ½ EcoRI          StuI                                    BamHi      ½ XbaI
    AA TTC-A GGC CTG CAC-CAT-CAC-CAT-CAT-CAC TAA GGATCC T
          G T CCG GAC GTG-GTA-GTG-GTA-GTA-GTG ATT CCTAGG AGATC
```

Un site Eco RI est ajouté en amont pour cloner les oligonucléotides appariés dans un vecteur pUC intermédiaire et pouvoir ainsi vérifier la séquence.

## Modification et clonage du CD4

### 1 - Séquençage de la région C-terminale du plasmide pGEM-T contenant le gène CD4 :

La région C-terminale du plasmide est vérifiée par séquençage, après une étape de PCR*.

### 2 - Addition de 6 résidus histidine en C-terminal du CD4 :

### 1- Amplification par PCR de la région Bsu361-Banim (dans le polylinker)

Oligonucléotide de PCR :

FOR-CD4 :

5'                                                    3'

CCT AAGCTG ATG CTG AGC TTG


BAC-CD4 :

BamHi       Pstl

5'                                                              3'

CAGT GGATCC AAT GGG GCT GCA GGT CTT CTG

*2- Addition de 6 codons His*


½ Pstl                                                    ½ BamHl

GC CCC ATT CAC CAT CAT CAC CAC CAT TTA G

ACGTCG GGG TAA GTG GTA GTA GTG GTG GTA ATT CCTAG


**Oligonucléotide de la PCR***

**CD4-HIS5**

5'                                                              3'

GCCCCATTCACCATCATCACCACCATTTAG


**CD4-HIS3**

3'                                                              5'

ACGTCGGGGGTAAGTGGTAGTAGTGGTGGTAATTCCTAG

5'                                                              3'

GATCCTTAATGGTGGTGATGATGGTGAATGGGGCTGCA


FOR-CD4 CCTAAGCTGATGCTGAGCTTG 40

BAC-CD4 CAGTGGATCCAATGGGGCTGCAGGTCTTCTG 40

CD4-HIS5 GCCCCATTCACCATCATCACCACCATTTAG 40

CD4-HIS3 GATCCTTAATGGTGGTGATGATGGTGAATGGGGCTGCA 40


Marquage avec la calcéine de cellules exprimant l'enveloppe de HIV-1


Réactifs :


- culture cellulaire
- calcéine AM (Molecular Probe, INC) 1µg/ml(~2,5 mM) (50µg de calcéine / 20 µl de DMSO, stocké à -20°C)
- DMSO (diméthyl sulfoxyde)
- RPMI ou DMEM
- PBS pH 7,4 (Dulb PBS de Gibco BRL)
- centrifugation avec IEC Central MO4R

Méthode :

1. Comptage des cellules dans le flacon.
2. Centrifugation à 1000rpm, pendant 5 min.
3. Remise en suspension des cellules dans 5 ml de milieu (0,5 x $10^6$ cellules/ml, par exemple).
4. Addition de 1μl de calcéine/5 ml de cellules ($\sim$ 2,5 mmoles de calcéine).
5. Vortex, afin d'obtenir un bon mélange.
6. Incubation pendant 45 min à 37°C, 5-7% de $CO_2$, à l'abri de la lumière.
7. Centrifugation à 1000 rpm pendant 5 min.
8. Lavage à 2 reprises avec 10 ml de milieu (ou PBS).
9. Remise en suspension des cellules dans 5 ml de milieu (ou PBS) ou à une concentration de 0,5 x $10^6$ cellules/ml.
10. Incubation 30 min à 37°C, 5-7% de $CO_2$, à l'abri de la lumière.
11. Lavage à 2 reprises avec 10 ml de milieu (ou PBS).
12. Remise en suspension des cellules dans 5 ml de milieu (ou PBS) ou à une concentration de 0,5-1 x $10^6$ cellules/ml Vortex.

Marquage des cellules cibles avec CMTMR

Le CMTMR est un dérivé fluorescent de chlorométhyle qui diffuse librement à travers les membranes des cellules vivantes. Une fois à l'intérieur de la cellule, la sonde légèrement thiol-réactive subit ce qui doit être une réaction médiée par la glutathione-S- transférase pour produire des produits d'addition colorants fluorescents imperméables à la membrane.
La coloration des cellules avec CMTMR donne une vive fluorescence qui résulte de la réaction avec les protéines dans les régions périnucléaires, ER et Golgi, qui sont immobiles, ainsi qu'à une plus faible fluorescence due au produit d'addition du glutathion fluorescent (PM $\sim$ 600 Da) dans le cytosol qui est capable de diffuser à travers les petits pores de fusion. 1. On solubilise la sonde fluorescente cytoplasmique CMTMR (5-(et -6-)-(((4-chlorométhyl) benzoyl) amino) tetraméthylrhodamine) (Moleculer Probes cat # C-2927, ex/em 541/565 nm) à une concentration de 10 mM dans DMSO.
On prélève des quantités aliquotes de 10 ou 20 μl, qu'on stocke à -20°C. On prépare une dilution de CMTMR (1:500) dans du DMEM supplémenté avec 10% de SVF inactivé par la chaleur 100 u/ml de pénicilline, 100 μg/ml de streptomycine (D10).
2. On élimine le milieu des cellules cibles (sur les micropuits) et on étale 1 ml d'une solution diluée de CMTMR. On met à incuber les échantillons à 37°C pendant 45-60 min. On remplace la solution colorante par 1ml de D10 et on continue l'incubation pendant 15 à 30 min de plus.

Conduite de la fusion

1. On ajoute des cellules effectrices marquées par la calcéine (2 ml) à des cellules cibles marquées par CMTMR. On fait incuber les deux populations cellulaires, 3 à 5 h à 37°C.
2. A la fin des incubations, on remplace le milieu par 1 ml de D-PBS et on prend les images de phase et de fluorescence avec des lentilles d'immersion dans l'huile 40 x.
Pour observer les cellules colorées avec la calcéine, on utilise le FITC (excitateur : BP 470-490 ; prisme DM 505; émetteur : BA 515-550) et pour CMTMR, on utilise de la rhodamine (excitateur : BP 530-550, prisme DM 570, émetteur BA 590). On évite ainsi le débordement qui se produit lorsqu'on observe la fluorescence des colorants. On réunit les images avec 6-10 filtres différents choisis au hasard pour chaque échantillon.
3. On analyse les données en utilisant le logiciel Metamorph (Universal Imaging Inc.), en superposant et en comptant les images. On compte le nombre total de cellules positives pour CMTMR et celles positives pour les 2 sondes fluorescentes. On utilise des images en champ brillant pour distinguer les taux positifs où les cellules marquées se recouvrent les uns les autres, mais n'ont pas fusionné. On calcule le pourcentage de fusion comme suit :

$$\% \text{ fusion} = 100 \times \frac{[\text{nombre de cellules positives pour les 2 colorants}]}{[\text{nombre total de cellules cibles}]}$$

**Revendications**

1. Compositions immunogènes, **caractérisées en ce qu'**elles sont élaborées à partir de préparations obtenues par

   . incubation de premiers moyens exprimant le et/ou les récepteurs cibles d'un agent pathogène infectieux, provoquant des infections chez un mammifère par liaison puis fusion avec des cellules cibles, avec des seconds moyens exprimant au moins les régions de l'agent pathogène reconnaissant lesdites cibles, dans des conditions permettant l'interaction des premiers et seconds moyens de manière à former un complexe, cette étape d'incubation étant réalisée selon des durées différentes, afin de conduire à des complexes correspondant à différents stades de fusion, et

   . mise en contact des complexes formés avec un agent fixateur, pendant des durées différentes, de manière à fixer des complexes avec différentes expositions et conformations des épitopes contre lesquels on souhaite former des anticorps,

      lesdits premiers et seconds moyens étant tolérés par les mammifères.

2. Compositions selon la revendication 1, **caractérisées en ce que** les premiers moyens sont des cellules autologues de mammifères, en particulier des cellules humaines saines prélevées chez un patient à vacciner.

3. Compositions selon la revendication 1, **caractérisées en ce que** les premiers moyens sont des vecteurs exprimant le ou les récepteurs cibles à leur surface.

4. Compositions selon la revendication 1, **caractérisées en ce que** les premiers moyens sont des liposomes portant le ou les récepteurs cibles à leur surface, notamment portant à leur surface les vecteurs tels que définis dans la revendication 3.

5. Compositions selon l'une quelconque des revendications 1 à 4, **caractérisées en ce que** les seconds moyens sont des cellules préalablement transformées par un vecteur portant au moins une région de liaison à au moins un récepteur, notamment par un vecteur viral.

6. Compositions selon l'une quelconque des revendications 1 à 4 **caractérisées en ce que** les seconds moyens sont constitués par des vecteurs viraux portant au moins ne région de liaison à au moins un récepteur cible.

7. Compositions selon l'une quelconque des revendications 1 à 4, **caractérisées en ce que** les seconds moyens sont des cellules infectées produisant les agents pathogènes ou sont constitués par les agents pathogènes infectieux.

8. Compositions selon l'une quelconque des revendications 1 à 7, **caractérisées en ce que** lesdits agents pathogènes sont des virus, et notamment des rétrovirus, des bactéries, des mycobactéries, ou des parasites comme *Plasmodium sp, Leishmania sp, Trypanosoma cruzi* et *Trypanosoma brucei.*

9. Compositions selon la revendication 8, **caractérisées en ce que** l'agent pathogène est VIH.

10. Compositions selon l'une quelconque des revendications 1 à 7 en combinaison avec la revendication 9, **caractérisées en ce que** lesdites préparations sont obtenues par incubation de premiers moyens exprimant le récepteur CD4 et/ou des co-récepteurs du VIH, avec des seconds moyens exprimant au moins les régions conservées des protéines d'enveloppe gp120 ou gp160.

11. Compositions selon la revendication 10, **caractérisées en ce que** les premiers moyens mis en oeuvre sont constitués par des cellules autologues de mammifères stimulées de manière à exprimer, en quantité suffisante pour l'interaction recherchée, le récepteur CD4 et/ou des co-récepteurs de VIH.

12. Compositions selon la revendication 10, **caractérisées en ce que** les premiers moyens sont des vecteurs viraux exprimant à leur surface CD4 et/ou des co-récepteurs de VIH, tels que le baculovirus, le virus de la forêt de Semliki ou encore des levures comme *Saccharomyces cerevisae.*

13. Compositions selon la revendication 10, **caractérisées en ce que** les premiers moyens sont des liposomes exprimant à leur surface le récepteur CD4 et/ou des co-récepteurs du VIH, ladite expression étant assurée par des vecteurs viraux.

14. Compositions selon l'une quelconque des revendications 10 à 13, **caractérisées en ce que** les seconds moyens sont constitués par des cellules préalablement transformées avec un vecteur viral comportant au moins les régions conservées des protéines d'enveloppe gp120 ou gp160, ou sont constitués par de tels vecteurs viraux.

15. Compositions selon l'une quelconque des revendications 10 à 13, **caractérisées en ce que** les seconds moyens sont des cellules infectées produisant VIH ou sont constitués par le virus VIH lui-même,

16. Compositions selon l'une quelconque des revendications 10 à 13, **caractérisées en ce que** les seconds moyens sont constitués par les protéines d'enveloppe gp120 ou gp160, sous forme naturelle ou recombinante, ou par au moins les régions conservées de telles protéines.

17. Compositions selon l'une quelconque des revendications 10 à 16, **caractérisées en ce que** l'un des co-récepteurs du VIH est remplacé par un anticorps monoclonal.

18. Compositions selon la revendication 17, **caractérisées en ce qu'**elles comprennent comme premier moyen du CD4 soluble et comme second moyen de la gp120 soluble, le cas échéant sous forme recombinante, ainsi qu'un anticorps monoclonal dirigé contre la zone de la gp120 qui se fixe sur les co-récepteurs.

19. Compositions selon l'une quelconque des revendications 1 à 18, **caractérisées en ce que** les préparations, après incubation, sont fixées avec de l'aldidrithiol-2.

20. Sérums et anticorps formés contre les compositions selon l'une quelconque des revendications 1 à 19.

21. Compositions vaccinales, contre une pathologie infectieuse, destinées à un mammifère, **caractérisées en ce qu'**elles renferment une quantité efficace d'une composition immunogène selon l'une quelconque des revendications 1 à 19, avec un véhicule inerte, acceptable pour l'administration à un mammifère, avec le cas échéant un adjuvant.

## Claims

1. Immunogenic compositions, **characterized in that** they are prepared from preparations obtained by

   - incubation of first means expressing the target receptor(s) of an infectious pathogenic agent, causing infections in a mammal by linkage then fusion with the target cells, with second means expressing at least the regions of the pathogenic agent recognizing said targets, in conditions allowing the interaction of the first and second means so as to form a complex, this incubation stage being realized for different periods of time in order to lead to complexes corresponding to different fusion stages, and
   - establishing contact between the complexes formed and a fixing agent, for different periods of time, so as to fix complexes with different exposures and conformations of the epitopes against which it is desired to form antibodies,
   - said first and second means being tolerated by mammals.

2. Compositions according to claim 1, **characterized in that** the first means are autologous cells of mammals, in particular healthy human cells taken from a patient to be vaccinated.

3. Compositions according to claim 1, **characterized in that** the first means are vectors expressing the target receptor(s) on their surface.

4. Compositions according to claim 1, **characterized in that** the first means are liposomes carrying the target receptor(s) on their surface, in particular carrying on their surface the vectors as defined in claim 3.

5. Compositions according to any one of claims 1 to 4, **characterized in that** the second means are cells previously transformed by a vector carrying at least one region linking to at least one receptor, in particular by a viral vector.

6. Compositions according to any one of claims 1 to 4, **characterized in that** the second means are constituted by viral vectors carrying at least one region linking to at least one target receptor.

7. Compositions according to any one of claims 1 to 4, **characterized in that** the second means are infected cells producing the pathogenic agents or are constituted by the infectious pathogenic agents.

8. Compositions according to any one of claims 1 to 7, **characterized in that** said pathogenic agents are viruses, and in particular retroviruses, bacteria, microbacteria or parasites such as *Plasmodium sp, Leishmania sp, Trypanosoma cruzi* and *Trypanosoma brucei*.

9. Compositions according to claim 8, **characterized in that** the pathogenic agent is HIV.

10. Compositions according to any one of claims 1 to 7 in combination with claim 9, **characterized in that** said preparations are obtained by incubation of first means expressing the HIV CD4 receptor and/or co-receptors, with second means expressing at least the preserved regions of envelope proteins gp120 or gp160.

11. Compositions according to claim 10, **characterized in that** the first means used are constituted by autologous cells of mammals stimulated so as to express, in a quantity sufficient for the sought interaction, the HIV CD4 receptor and/or co-receptors.

12. Compositions according to claim 10, **characterized in that** the first means are viral vectors expressing on their surface HIV CD4 and/or co-receptors such as baculovirus, the Semliki Forest virus or yeasts such as *Saccharomyces cerevisae*.

13. Compositions according to claim 10, **characterized in that** the first means are liposomes expressing on their surface the HIV CD4 receptor and/or co-receptors, said expression being provided by viral vectors.

14. Compositions according to any one of claims 10 to 13, **characterized in that** the second means are constituted by cells previously transformed with a viral vector carrying at least the preserved regions of envelope proteins gp120 or gp160, or are constituted by such viral vectors.

15. Compositions according to any one of claims 10 to 13, **characterized in that** the second means are infected cells producing HIV or are constituted by the HIV virus itself.

16. Compositions according to any one of claims 10 to 13, **characterized in that** the second means are constituted by the envelope proteins gp120 or gp160, in natural or recombinant form, or by at least the preserved regions of such proteins.

17. Compositions according to any one of claims 10 to 16, **characterized in that** one of the HIV co-receptors is replaced by a monoclonal antibody.

18. Compositions according to claim 17, **characterized in that** they comprise as first means soluble CD4 and as second means soluble gp120, if necessary in recombinant form, as well as a monoclonal antibody directed against the zone of the gp120 which fixes on the co-receptors.

19. Compositions according to any one of claims 1 to 18, **characterized in that** the preparations, after incubation, are fixed with aldidrithiol-2.

20. Serums and antibodies formed against the compositions according to any one of claims 1 to 19.

21. Vaccine compositions, against an infectious pathology, intended for a mammal, **characterized in that** they contain an effective quantity of an immunogenic composition according to any one of claims 1 to 19, with an inert vehicle, acceptable for administration to a mammal, if necessary with an adjuvant.

**Patentansprüche**

1. Immunogene Zusammensetzungen, **dadurch gekennzeichnet, daß** sie gewonnen sind aus Präparaten, die erhalten wurden durch

- Inkubation von ersten Mitteln, welche den Zielrezeptor oder die Zielrezeptoren eines infektiösen, pathogenen Agens exprimieren, der oder die bei einem Säugetier Infektionen durch Bindung an und anschließende Verschmelzung mit Zielzellen bewirken, mit zweiten Mitteln, die mindestens die Regionen des pathogenen Agens exprimieren, welche diese Ziele erkennen, unter Bedingungen, welche die Interaktion der ersten und zweiten Mittel zur Bildung eines Komplexes ermöglichen, wobei dieser Inkubationsstufe über Zeiten von verschieden langer Dauer durchgeführt wird, um zu Komplexen zu führen, die verschiedenen Stadien der Fusion entsprechen, und
- Inkontaktbringen der gebildeten Komplexe mit einem Fixationsmittel während verschieden langer Zeiten, um Komplexe mit verschiedenen Expositionen und Konformationen der Epitope zu fixieren, gegen welche man Antikörper bilden möchte,

wobei die ersten und zweiten Mittel von den Säugetieren toleriert werden.

2. Zusammensetzungen nach Anspruch 1, **dadurch gekennzeichnet, daß** die ersten Mittel autologe Zellen von Säugetieren sind, besonders gesunde humane Zeilen, die von einem zu impfenden Patienten entnommen wurden.

3. Zusammensetzungen nach Anspruch 1, **dadurch gekennzeichnet, daß** die ersten Mittel Vektoren sind, welche den oder die Zielrezeptoren an ihre Oberfläche exprimieren.

4. Zusammensetzungen nach Anspruch 1, **dadurch gekennzeichnet, daß** die ersten Mittel Liposome sind, welche den Zielrezeptor oder die Zielrezeptoren an ihrer Oberfläche tragen, besonders an ihrer Oberfläche die Vektoren wie im Anspruch 3 definiert tragen

5. Zusammensetzungen nach einem der Ansprüche 1 bis 4, **dadurch gekennzeichnet, daß** die zweiten Mittel Zellen sind, die zuvor durch einen Vektor transformiert wurden, der mindestens eine Bindungsregion mit mindestens einem Rezeptor trägt, besonders ein viraler Vektor.

6. Zusammensetzungen nach einem der Ansprüche 1 bis 4, **dadurch gekennzeichnet, daß** die zweiten Mittel aus viralen Vektoren bestehen, die mindestens eine Region der Bindung an mindestens einen Zielrezeptor tragen.

7. Zusammensetzungen nach einem der Ansprüche 1 bis 4, **dadurch gekennzeichnet, daß** die zweiten Mittel infizierte Zellen sind, welche die pathogenen Agenzien produzieren oder aus den infektiösen pathogenen Agenzien bestehen.

8. Zusammensetzungen nach einem der Ansprüche 1 bis 7, **dadurch gekennzeichnet, daß** die pathogenen Agenzien Viren und besonders Retroviren, Bakterien, Mikrobakterien oder Parasiten, wie *Plasmodium sp.*, *Leishmania sp. Trypanosoma cruzi* und *Trypanosoma brucei* sind.

9. Zusammensetzungen nach Anspruch 8, **dadurch gekennzeichnet, daß** das pathogene Agens VIH ist.

10. Zusammensetzungen nach einem der Ansprüche 1 bis 7 in Kombination mit Anspruch 9, **dadurch gekennzeichnet, daß** die Präparate erhalten werden durch Inkubation von ersten Mitteln, welche den Rezeptor CD4 und/oder Co-Rezeptoren des VIH exprimieren, mit zweiten Mitteln, welche mindestens die konservierten Bereiche der Hüllproteine gp120 oder gp160 exprimieren.

11. Zusammensetzungen nach Anspruch 10, **dadurch gekennzeichnet, daß** die eingesetzten ersten Mittel aus autologen Säugetierzellen bestehen, die so stimuliert sind, daß sie den Rezeptor CD4 und/oder Co-Rezeptoren von VIH in genügender Menge für die gewünschte Interaktion exprimieren.

12. Zusammensetzungen nach Anspruch 10, **dadurch gekennzeichnet, daß** die ersten Mittel virale Vektoren sind, die an ihrer Oberfläche CD4 und/oder Co-Rezeptoren von VIH exprimieren, wie der *Baculovirus,* der *Semliki-Forest-Virus* oder auch Hefen, wie *Saccharomyces cerevisae.*

13. Zusammensetzungen nach Anspruch 10, **dadurch gekennzeichnet, daß** die ersten Mittel Liposome sind, welche an ihrer Oberfläche den Rezeptor CD4 und/oder Co-Rezeptoren von VIH exprimieren, wobei die Expression durch virale Vektoren gewährleistet wird.

**14.** Zusammensetzungen nach einem der Ansprüche 10 bis 13, **dadurch gekennzeichnet, daß** die zweiten Mittel aus Zellen bestehen, die zuvor mit einem viralen Vektor transformiert wurden, der mindestens die konservierten Bereiche der Hüllproteine gp120 oder gp160 aufweist, oder aus solchen viralen Vektoren bestehen.

**15.** Zusammensetzungen nach einem der Ansprüche 10 bis 13, **dadurch gekennzeichnet, daß** die zweiten Mittel infizierte Zellen sind, welche VIH produzieren oder aus dem VIH Virus selbst bestehen.

**16.** Zusammensetzungen nach einem der Ansprüche 10 bis 13, **dadurch gekennzeichnet, daß** die zweiten Mittel aus den Hüllproteinen gp 120 oder gp160 in natürlicher oder rekombinanter Form oder aus mindestens den konservierten Bereichen solcher Proteine bestehen.

**17.** Zusammensetzungen nach einem der Ansprüche 10 bis 16, **dadurch gekennzeichnet, daß** der eine der Co-Rezeptoren des VIH ersetzt ist durch einen monoklonalen Antikörper.

**18.** Zusammensetzungen nach Anspruch 17, **dadurch gekennzeichnet, daß** sie als erstes Mittel lösliches CD4 und als zweites Mittel lösliches gp120 aufweisen, gegebenenfalls in rekombinanter Form, sowie einen monoklonalen Antikörper, der gegen den Bereich des gp120 gerichtet ist, der sich an den Co-Rezeptoren fixiert.

**19.** Zusammensetzungen nach einem der Ansprüche 1 bis 18, **dadurch gekennzeichnet, daß** die Präparate nach der Inkubation mit Aldidrithiol-2 fixiert werden.

**20.** Seren und Antikörper, die gegen die Zusammensetzungen nach einem der Ansprüche 1 bis 19 gebildet sind.

**21.** Impfstoffzusammensetzungen gegen eine infektiöse Pathologie, die für eine Säugetier bestimmt sind, **dadurch gekennzeichnet, daß** sie eine wirksame Menge einer immunogenen Zusammensetzung gemäß einem der Ansprüche 1 bis 19 mit einem inerten Vehikel, das für die Verabreichung an ein Säugetier annehmbar ist, gegebenenfalls mit einem Hilfsmittel umfassen.